# EUROPEAN PATENT APPLICATION

(11) **EP 4 682 155 A1**
(43) Date of publication of application: **21.01.2026**
(21) Application number: 24771009.8
(22) Date of filing: 15.03.2024
(51) Int. Cl.: C07H 23/00, C07H 19/173

(54) **PRODUCTION METHODS FOR 2'-O-MODIFIED ADENOSINE PHOSPHORAMIDITE AND INTERMEDIATE THEREOF**

(30) Priority: 15.03.2023 JP 2023040938
(71) Applicant: Nissan Chemical Corporation, Tokyo 103-6119 (JP)
(72) Inventor: NAKANO, Satoshi, Funabashi-shi, Chiba 274-8507 (JP); YAMAMOTO, Akio, Funabashi-shi, Chiba 274-8507 (JP)
(74) Representative: Hoffmann Eitle
(86) International application number: PCT/JP2024/010287
(87) International publication number: WO 2024/190907

(57) **Abstract**

The present invention provides a novel method for producing 2'-O-modified adenosine phosphoramidite represented by formula (7) and intermediates thereof. 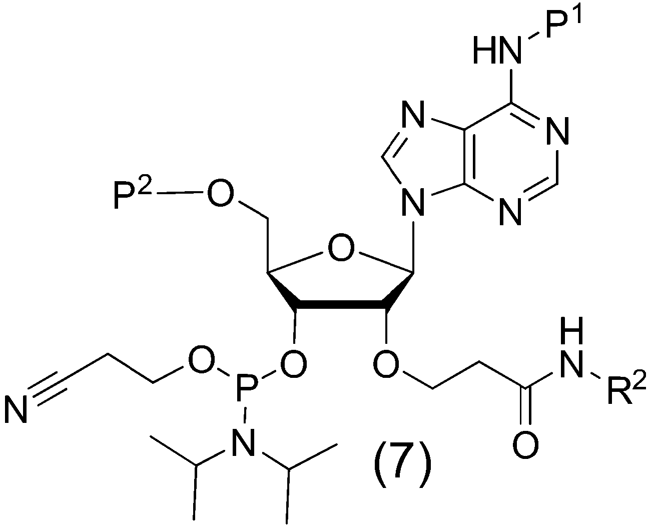 In the formula,
R² represents an optionally substituted C₁₋₆ alkyl group, an optionally substituted C₇₋₁₆ aralkyl group, an optionally substituted C₂₋₆ alkenyl group, an optionally substituted C₂₋₆ alkynyl group, or an optionally substituted C₆₋₁₄ aryl group;
P¹ represents a protecting group of an amino group; and
P² represents a protecting group of a hydroxy group.

## Description

### TECHNICAL FIELD

The present invention relates to novel methods for producing a 2'-O-modified adenosine phosphoramidite and an intermediate thereof.

### BACKGROUND ART

Nucleic acid drugs are therapeutic agents composed of nucleic acids (oligonucleotides) that form complementary base pairs with target DNA or RNA, and are expected to serve as novel therapeutic agents. Thus, as nucleic acid units for use in nucleic acid drugs, various artificial nucleic acid units (artificial nucleosides or artificial nucleotides with phosphate groups bonded thereto) have been developed by modifying the structures of natural nucleic acids. For example, nucleosides in which the 2'-oxygen atom of the sugar moiety of a ribonucleoside is methylated, 2-methoxyethylated, or 2-(N-methylcarbamoyl)ethylated have been reported (for example, refer to Patent Documents 1 and 2 and Non-Patent Document 1). In particular, oligonucleotides containing 2'-O-2-(N-methylcarbamoyl)ethylated nucleosides have been reported to exhibit improved resistance to nucleases, which are degradative enzymes present in vivo, compared to oligonucleotides containing 2'-O-methylated nucleosides (for example, refer to Non-Patent Document 1).

It has been reported that 2'-O-2-(N-substituted carbamoyl)ethylated nucleosides such as the 2'-O-2-(N-methylcarbamoyl)ethylated nucleosides can be produced through a Michael addition reaction between an acrylic acid ester and a ribonucleoside, followed by amidation or other reactions (for example, refer to Patent Document 2 and Non-Patent Document 1).

### Prior Art Documents

### Patent Documents

Patent Document 1: Japanese Unexamined Patent Application Publication No. 7-2889 A
Patent Document 2: Japanese Patent No. 5194256

### Non-Patent Documents

Non-Patent Document 1: Journal of Organic Chemistry, vol. 76, p. 3042 (2011)

### SUMMARY OF THE INVENTION

### Problem to be Solved by the Invention

In the production methods described in Patent Document 2 and Non-Patent Document 1, cesium carbonate is used as a base in the Michael addition reaction step. Cesium carbonate is a solid that is highly hygroscopic and poorly soluble in organic solvents, resulting in large differences between manufacturers and lots, and consequently, the Michael addition reaction exhibits low reproducibility. In addition, the method involves many steps requiring complex purification by column chromatography, which is unsuitable for an industrial production method.

The present invention provides a novel method for producing a 2'-O-2-(N-substituted carbamoyl)ethylated adenosine phosphoramidite and an intermediate thereof, which is useful as an industrial production method.

### Means for Solving the Problem

The inventors have conducted extensive studies and have, for the first time, found that: (1) the use of a catalytic amount of an alkali metal tertiary alkoxide improves the reproducibility of the Michael addition reaction; (2) the product of the amide formation step can be purified by a precipitation operation and solid-liquid separation; (3) among three steps-the adenine protection step, the silyl protecting group removal step, and the subsequent step of protecting the 5'-hydroxy group of the ribose moiety-, purification by column chromatography in the first two steps can be omitted; and (4) when the purification by column chromatography in the phosphoramidite formation step is performed using diol silica gel, the operations, materials and time required for purification can be saved. Based on the above findings, the inventors conducted further studies, and completed a method for producing the adenosine phosphoramidite that is suitable for industrial production.

Specifically, the present invention relates to the following aspects.
[1] A method for producing a compound of Formula (2), characterized by comprising adding a catalytic amount of an alkali metal tertiary alkoxide to a solution or suspension of a mixture of a compound of the following Formula (1) and an acrylic acid ester of Formula (8) in a tertiary alcohol-based solvent: 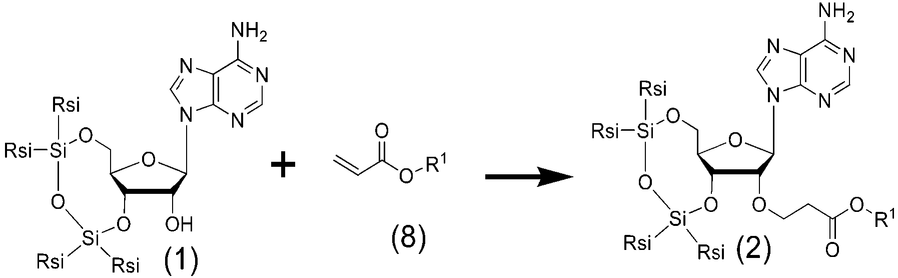 [wherein R¹ is a C₁₋₄ alkyl group which arbitrarily has a substituent or a C₇₋₁₆ aralkyl group which arbitrarily has a substituent, and Rsi's are each independently a C₁₋₆ alkyl group or a phenyl group, and a symbol + indicates that the compound of Formula (1) and the compound of Formula (8), which are described on the left and right of +, respectively, are mixed together].
[2] The method according to [1], wherein R¹ is a methyl group, an ethyl group, a trifluoroethyl group, or a benzyl group.
[3] The method according to [1], wherein R¹ is a methyl group.
[4] The method according to any one of [1] to [3], wherein the tertiary alcohol-based solvent is tert-butanol.
[5] The method according to any one of [1] to [4], wherein the number of equivalents of the alkali metal tertiary alkoxide per 1 equivalent of the compound of Formula (1) is 0.1 equivalents to 0.8 equivalents.
[6] The method according to any one of [1] to [5], wherein the alkali metal tertiary alkoxide is potassium tert-butoxide.
[7] A method for producing a compound of the following Formula (3), comprising:
   a step (b1) of reacting a compound of the following Formula (2) with a primary amine of the following Formula (9) to produce a compound of the following Formula (3); and
   a step (b2) of purifying the compound of Formula (3) by a precipitation operation and solid-liquid separation: 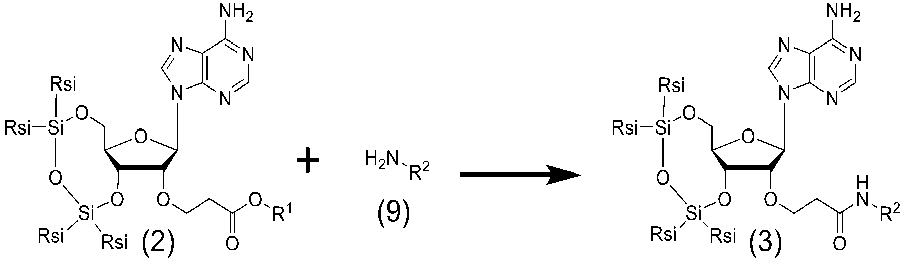
   [in Formula (2), R¹ is a C₁₋₄ alkyl group which arbitrarily has a substituent or a C₇₋₁₆ aralkyl group which arbitrarily has a substituent,
   in Formulae (2) and (3), Rsi's are each independently a C₁₋₆ alkyl group or a phenyl group, and
   in Formulae (3) and (9), R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent, and a symbol + indicates that the compound of Formula (2) and the compound of Formula (9), which are described on the left and right of +, respectively, are mixed together].
[8] The method according to [7], wherein R¹ is a methyl group, an ethyl group, a trifluoroethyl group, or a benzyl group.
[9] The method according to [7], wherein R¹ is a methyl group.
[10] The method according to any one of [7] to [9], wherein R² is a methyl group.
[11] A method for producing a compound of the following Formula (6), comprising:
   a step (c) of converting a compound of the following Formula (3) into a compound of the following Formula (4);
   a step (d) of converting the compound of Formula (4) into a compound of the following Formula (5); and
   a step (e) of converting the compound of Formula (5) into a compound of the following Formula (6): 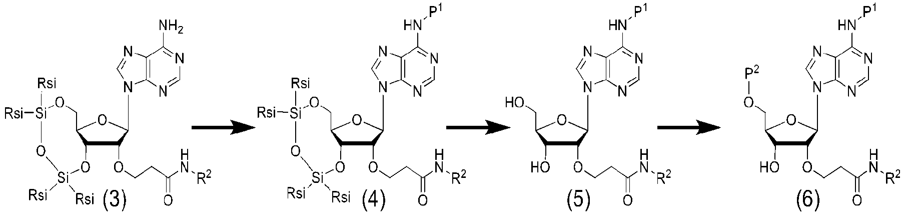
   [in Formulae (3), (4), (5), and (6), R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent,
   in Formulae (3) and (4), Rsi's are each independently a C₁₋₆ alkyl group or a phenyl group,
   in Formulae (4), (5), and (6), P¹ is a protecting group for an amino group, and in Formula (6), P² is a protecting group for a hydroxy group].
[12] The method according to [11], wherein R² is a methyl group.
[13] The method according to [11] or [12], wherein P¹ is an acetyl group, a propionyl group, an i-butyryl group, a pivaloyl group, an n-butyryl group, a benzoyl group, or a phenoxyacetyl group.
[14] The method according to [11] or [12], wherein P¹ is an acetyl group.
[15] The method according to any one of [11] to [14], wherein P² is a 4,4'-dimethoxytrityl group.
[16] The method according to any one of [11] to [15], wherein the process proceeds to a next step without purification by column chromatography in the step (c) and the step (d), and purification by column chromatography is performed in the step (e).
[17] A method for purifying a compound of the following Formula (7) by column chromatography using a diol silica gel: 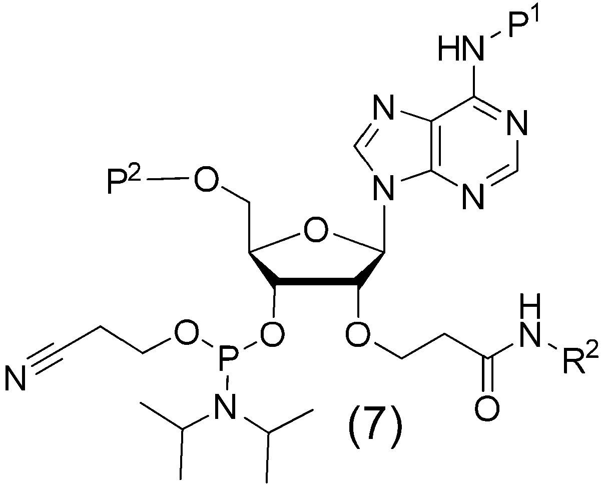 [wherein R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent, P¹ is a protecting group for an amino group, and P² is a protecting group for a hydroxy group].
[18] The method according to [17], wherein R² is a methyl group.
[19] The method according to [17] or [18], wherein P¹ is an acetyl group, a propionyl group, an i-butyryl group, a pivaloyl group, an n-butyryl group, a benzoyl group, or a phenoxyacetyl group.
[20] The method according to [17] or [18], wherein P¹ is an acetyl group.
[21] The method according to any one of [17] to [20], wherein P² is a 4,4'-dimethoxytrityl group.
[22] A method for producing a compound of Formula (7), comprising:
   producing a compound of Formula (6) by the method according to [11];
   producing the compound of Formula (7) by converting the compound of Formula (6) into a phosphoramidite; and
   purifying the compound of Formula (7) by the method according to [17].
[23] The method according to [22], wherein the compound of Formula (3) is produced by the method according to [7].
[24] The method according to [23], wherein the compound of Formula (2) is produced by the method according to [1].

### Effects of the Invention

According to the present invention, it is possible to provide a novel method for producing a 2'-O-2-(N-substituted carbamoyl)ethylated adenosine phosphoramidite. According to the present invention, in the production of a 2'-O-2-(N-substituted carbamoyl)ethylated adenosine phosphoramidite using an adenosine compound in which the 3'- and 5'-hydroxy groups are protected as a starting material, the effort required for purification by column chromatography can be significantly reduced. Therefore, the present invention makes it possible to industrially produce the 2'-O-2-(N-substituted carbamoyl)ethylated adenosine phosphoramidite and makes it possible to produce the compound on a large scale.

In addition, according to the present invention, it is possible to obtain a high-purity 2'-O-2-(N-substituted carbamoyl)ethylated adenosine phosphoramidite.

### MODES FOR CARRYING OUT THE INVENTION

Hereinafter, the present invention will be described in more detail. Here, in the present invention, "n-" means normal, "i-" means iso, "s-" and "sec-" mean secondary, "t-" and "tert-" mean tertiary, "o-" means ortho, "m-" means meta, "p-" means para, "Ph" means phenyl, "Bu" means butyl, "Ts" means p-toluenesulfonyl, and "Bn" means benzyl.

First, the terms used in the description of chemical structures in this specification will be described.

The term "C₁₋₆ alkyl group" refers to a linear or branched alkyl group having 1 to 6 carbon atoms, and specific examples thereof include a methyl group, ethyl group, n-propyl group, i-propyl group, n-butyl group, i-butyl group, s-butyl group, t-butyl group, n-pentyl group, 2-methylbutyl group, 3-methylbutyl group, n-hexyl group, 2-methylpentyl group, 3-methylpentyl group, 4-methylpentyl group, 2-ethylbutyl group, and 3-ethylbutyl group.

The term "C₁₋₄ alkyl group" refers to a linear or branched alkyl group having 1 to 4 carbon atoms, and specific examples thereof include a methyl group, ethyl group, n-propyl group, i-propyl group, n-butyl group, i-butyl group, s-butyl group and t-butyl group.

The term "C₇₋₁₆ aralkyl group" refers to an alkyl group having an aromatic hydrocarbon as a substituent, and having a total of 7 to 16 carbon atoms in the entire substituent. Specific examples thereof include a phenylmethyl group (benzyl group), 1-phenylethyl group, 2-phenylethyl group, 1-phenylpropyl group, 2-phenylpropyl group, 3-phenylpropyl group, naphthalene-1-ylmethyl group, naphthalene-2-ylmethyl group, 2-(naphthalene-1-yl)ethyl group, 2-(naphthalene-2-yl)ethyl group, anthracene-1-ylmethyl group, anthracene-2-ylmethyl group, and anthracene-9-ylmethyl group.

The term "C₂₋₆ alkenyl group" refers to a linear or branched alkenyl group having 2 to 6 carbon atoms and at least one double bond, and specific examples thereof include an ethenyl group, 1-propenyl group, 2-propenyl group, 1-butenyl group, 2-butenyl group, 3-butenyl group, 1-pentenyl group, 2-pentenyl group, 3-pentenyl group, and 4-pentenyl group.

The term "C₂₋₆ alkynyl group" refers to a linear or branched alkynyl group having 2 to 6 carbon atoms and at least one triple bond, and specific examples thereof include an ethynyl group, 1-propynyl group, 2-propynyl group, 1-butynyl group, 2-butynyl group, 3-butynyl group, 1-pentynyl group, 2-pentynyl group, 3-pentynyl group, and 4-pentynyl group.

The term "C₆₋₁₄ aryl group" refers to an aromatic hydrocarbon group having 6 to 14 carbon atoms, and specific examples thereof include a phenyl group, 1-naphthyl group, 2-naphthyl group, 1-anthracenyl group, 2-anthracenyl group, and 9-anthracenyl group.

The term "which arbitrarily has a substituent" means that the group is unsubstituted or substituted with any number of optional substituents. The term "substituted" refers to being substituted with any number of optional substituents. Any number is, for example, 1 to 3. The "optional substituents" described above are not particularly limited in type as long as they do not adversely affect this reaction. Examples of optional substituents include halogen atoms, hydroxy groups, protected hydroxy groups, amino groups, protected amino groups, carboxyl groups, protected carboxyl groups, nitro groups, cyano groups, C₁₋₆ alkyl groups, C₂₋₆ alkenyl groups, C₆₋₁₄ aryl groups, and C₇₋₁₆ aralkyl groups, as well as phenyl groups substituted with these substituents.

The halogen atom is a fluorine atom, chlorine atom, bromine atom, or iodine atom.

The term "protecting group for a hydroxy group" refers to a protecting group generally used in organic synthetic chemistry (particularly nucleic acid synthesis). Examples thereof include C₁₋₆ alkyl groups (methyl group, ethyl group, tert-butyl group, etc.), allyl groups, benzyl-based protecting groups (benzyl group, p-methoxybenzyl group, etc.), acyl-based protecting groups (formyl group, acetyl group, benzoyl group, etc.), acetal-based protecting groups (methoxymethyl group, ethoxymethyl group, 2-tetrahydropyranyl group, etc.), silyl-based protecting groups (trimethylsilyl group, triisopropylsilyl group, t-butyldimethylsilyl group, t-butyldiphenylsilyl group, etc.), and triarylmethyl-type protecting groups (trityl group, 4,4'-dimethoxytrityl group, pixyl group, etc.), but the present invention is not limited thereto. Particularly preferable protecting groups for hydroxy groups in the steps will be described below.

The term "protecting group for an amino group" refers to a protecting group generally used in organic synthetic chemistry (particularly nucleic acid synthesis). Examples thereof include acyl-based protecting groups (formyl group, acetyl group, benzoyl group, i-butyryl group, pivaloyl group, trifluoroacetyl group, phenoxyacetyl group, etc.), carbamate-based protecting groups (tert-butoxycarbonyl group, benzyloxycarbonyl group, 9-fluorenylmethyloxycarbonyl group, etc.), sulfonyl-based protecting groups (methanesulfonyl group, p-toluenesulfonyl group, 2-nitrobenzenesulfonyl group, etc.), triarylmethyl-based protecting groups (trityl group, 4,4'-dimethoxytrityl group, etc.), and amidine-based protecting groups (N,N-dimethylaminomethylene group, N,N-diethylaminomethylene group, N,N-diisopropylaminomethylene group, 1-pyrrolidinomethylene group, etc.), but the present invention is not limited thereto. Particularly preferable protecting groups for amino groups in the steps will be described below.

The term "protecting group for a phosphate group" refers to a protecting group generally used in organic synthetic chemistry (particularly nucleic acid synthesis). Examples thereof include alkyl-based protecting groups (2-cyanoethyl group, 2,2,2-trichloroethyl group, and benzyl group), and aryl-based protecting groups (2-chlorophenyl group, and 4-chlorophenyl group), but the present invention is not limited thereto.

The term "deprotection" refers to removal of a protecting group that is generally used in the organic synthesis reaction. The protected hydroxy group, the protected amino group and the protected carboxyl group can be subjected to a deprotection reaction (for example, refer to Protective Groups in Organic Synthesis, Fourth edition, authored by T. W. Greene, and John Wiley & Sons Inc. (2006)) to remove their protecting groups.

The type of base used in this reaction is not limited as long as it does not affect the reaction, and examples thereof include triethylamine, N,N-diisopropylethylamine, N-methylmorpholine, pyridine, 1,8-diazabicyclo[5.4.0]-7-undecene, sodium carbonate, potassium carbonate, cesium carbonate, sodium hydroxide, potassium hydroxide, cesium hydroxide, sodium methoxide, potassium methoxide, sodium tert-butoxide, and potassium tert-butoxide. Particularly preferable bases in the steps will be described below.

The bases used may be used alone or two or more thereof may be used in combination.

The solvent used in the present invention is not limited as long as it does not inhibit the progress of the reaction, and examples of preferable solvents include amide-based solvents (for example, N,N-dimethylformamide, N,N-dimethylacetamide, N-methylpyrrolidone, etc.), urea-based solvents (tetramethyl urea, N,N-dimethylimidazolidinone, etc.), ether-based solvents (for example, diethyl ether, diisopropyl ether, t-butyl methyl ether, tetrahydrofuran, dioxane, dimethoxyethane, diethoxyethane, etc.), aliphatic hydrocarbon-based solvents (for example, n-pentane, n-hexane, n-heptane, cyclohexane, petroleum ether, etc.), aromatic hydrocarbon-based solvents (benzene, chlorobenzene, o-dichlorobenzene, nitrobenzene, toluene, xylene, mesitylene, tetralin, etc.), halogenated hydrocarbon-based solvents (for example, chloroform, dichloromethane, 1,2-dichloroethane, carbon tetrachloride, etc.), lower aliphatic acid ester-based solvents (for example, methyl acetate, ethyl acetate, isopropyl acetate, methyl propionate, etc.), alcohol-based solvents (for example, methanol, ethanol, n-propanol, i-propanol, n-butanol, tert-butanol, etc.), dimethyl sulfoxide, sulfolane, acetic acid, and water. Particularly preferable solvents in the steps will be described below.

The solvents may be used alone or two or more types thereof may be used in combination.

The term "precipitation operation" refers to an operation of causing a compound to precipitate from a solution containing the compound. Specific methods thereof include, for example, a method in which a compound is dissolved in a good solvent (a solvent in which the compound is highly soluble) to form a solution, and a poor solvent (a solvent in which the compound is less soluble) is then added to the solution and a method in which the temperature of a compound suspension is raised and the temperature of the solution is then lowered. The purity of the compound can be improved by the precipitation operation.

The term "solid-liquid separation" refers to an operation of separating a solid dispersed in a liquid, such as a solution, from the liquid. Examples of specific operations include filtration and centrifugation, and filtration is preferable.

Hereinafter, the present invention will be described in detail. In the drawings described below through the examples, R¹ and R², and P¹ and P² in the formulae have the same meanings as above unless otherwise specified.

A method for producing a series of compounds of the present invention is shown in the following scheme. In the scheme, "step" refers to a step. Here, "compound of Formula (formula number)" may be referred to as "compound (formula number)" in the detailed description below. For example, "compound of Formula (1)" is referred to as "compound (1)."

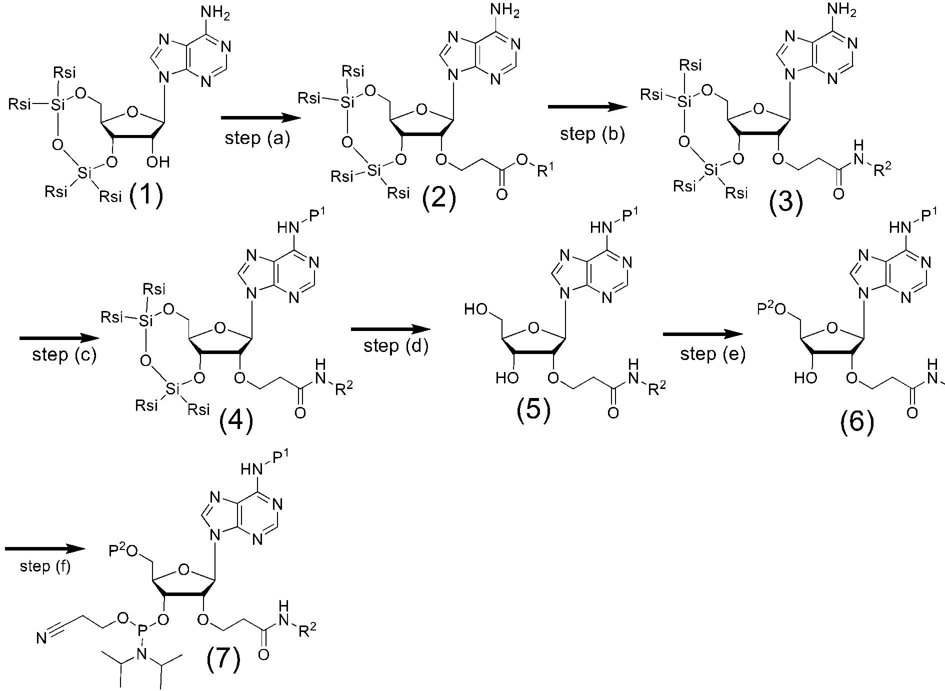

R¹ is a C₁₋₄ alkyl group which arbitrarily has a substituent or a C₇₋₁₆ aralkyl group which arbitrarily has a substituent, preferably a C₁₋₄ alkyl group which may have one or more halogen atoms or a C₇₋₁₆ aralkyl group which may have one or more halogen atoms, more preferably a methyl group, ethyl group, trifluoroethyl group or benzyl group, and particularly preferably a methyl group.

R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent, preferably a C₁₋₆ alkyl group, more preferably a C₁₋₄ alkyl group, and particularly preferably a methyl group.

Rsi's are each independently a C₁₋₆ alkyl group or a phenyl group, preferably a methyl group, i-propyl group or phenyl group, and particularly preferably an i-propyl group.

P¹ is a protecting group for an amino group, preferably an acetyl group, propionyl group, i-butyryl group, pivaloyl group, n-butyryl group, benzoyl group, or phenoxyacetyl group, and particularly preferably an acetyl group.

P² is a protecting group for a hydroxy group, preferably a triarylmethyl-based protecting group, and particularly preferably a 4,4'-dimethoxytrityl group.

Step (a): Michael addition reaction step

The compound (2) can be synthesized by reacting the 2'-hydroxy group of the compound (1) with an acrylic acid ester (the following Formula (8)). The compound (1) and the acrylic acid ester (8) are commercially available products or can be synthesized by methods well known to those skilled in the art.

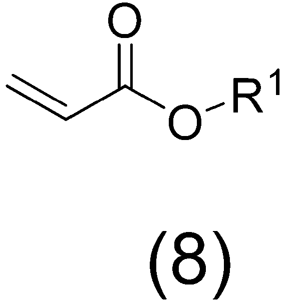

The substituent R¹ of the acrylic acid ester (8) that can be used in this reaction is preferably a C₁₋₄ alkyl group which arbitrarily has a substituent or a C₇₋₁₆ aralkyl group which arbitrarily has a substituent, more preferably a C₁₋₄ alkyl group or C₇₋₁₆ aralkyl group which may have one or more halogen atoms, still more preferably a methyl group, ethyl group, 2,2,2-trifluoroethyl group, n-butyl group, i-butyl group, or benzyl group, and particularly preferably a methyl group.

The amount of the acrylic acid ester used varies depending on the type of the substituent R¹, but is preferably 1 to 100 mol, more preferably 5 to 50 mol equivalents, still more preferably 10 to 30 mol equivalents, and particularly preferably 10 to 20 mol equivalents, per mol equivalent of the compound (1).

The solvent used in this reaction is preferably a tertiary alcohol-based solvent (tert-butanol, tert-pentyl alcohol, etc.) and more preferably tert-butanol. The compound (1) and the acrylic acid ester (8) may be completely dissolved in the solvent to form a solution, or may form a suspension without being completely dissolved. It is preferable that the compound (1) and the acrylic acid ester (8) be completely dissolved in the solvent to form a solution.

In this reaction, it is preferable to use a base. The type of base used in this reaction is preferably an alkali metal tertiary alkoxide (sodium tert-butoxide, potassium tert-butoxide, sodium tert-pentoxide, etc.), and more preferably potassium tert-butoxide.

The amount of base used is preferably a catalytic amount, and is preferably 0.1 to 1.0 mol equivalents, more preferably 0.1 to 0.8 mol equivalents, and still more preferably 0.1 to 0.5 mol equivalents, per 1 mol equivalent of the compound (1).

The base is preferably added to a solution or suspension of a mixture of the compound (1) and the acrylic acid ester (8), and more preferably added to the solution in consideration of convenience in production.

The base may be added as a solution by dissolving it in a solvent, may be added as a suspension by suspending it in a solvent, or may be added in the form of a solid. In consideration of convenience in production, it is more preferable to add the base as a solution by dissolving it in a solvent.

When the base is dissolved in a solvent or suspended in a solvent, the solvent is not limited as long as it does not inhibit the progress of the reaction, and tertiary alcohol-based solvents (tert-butanol, tert-pentyl alcohol, etc.) and ether-based solvents (for example, diethyl ether, diisopropyl ether, t-butyl methyl ether, tetrahydrofuran, dioxane, dimethoxyethane, diethoxyethane, etc.) are preferable, tert-butanol and tetrahydrofuran are more preferable, and tetrahydrofuran is still more preferable.

The reaction temperature is preferably 0 to 40°C and more preferably 0 to 30°C.

The reaction time is, for example, 10 minutes to 48 hours, preferably 10 minutes to 24 hours, and more preferably 10 minutes to 2 hours.

Step (b): amide formation step

The compound (3) can be synthesized by reacting the ester group of the compound (2) with a primary amine. The primary amine is expressed by the following Formula (9). 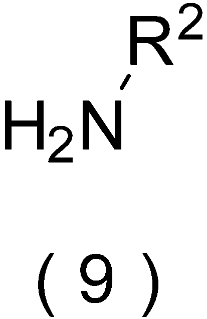 [wherein R² is as defined above]

In the present invention, the step (b) includes the following step (b1) and step (b2).

The step (b1) is a process of reacting the compound of Formula (2) with the primary amine of Formula (9) to synthesize a compound of Formula (3).

The step (b2) is a process of causing the compound of Formula (3) to precipitate from an organic solvent solution of a mixture containing the compound and performing isolation by solid-liquid separation.

### Step (b1)

The amount of the primary amine used varies depending on the type, and is preferably 1.0 to 100 mol equivalents, more preferably 5.0 to 50 mol equivalents, and still more preferably 10 to 30 mol equivalents, per 1 mol equivalent of the raw material compound (2). It is also possible to proceed to the step (b) without measuring the yield of the compound (2) obtained in the step (a), and in this case, the number of equivalents of the compound (2) can be replaced with the number of equivalents of the compound (1), which is a precursor of the compound (2).

The solvent used in this reaction is preferably an ether-based solvent or an alcohol-based solvent, and more preferably tetrahydrofuran or methanol.

The reaction temperature is preferably 0°C to the solvent reflux temperature, and more preferably 20 to 40°C.

The reaction time is, for example, 0.5 to 48 hours, preferably 1 to 36 hours, and still more preferably 4 to 24 hours.

### Step (b2)

The compound (3) can be obtained as a solid by the precipitation operation and the solid-liquid separation.

The solvent used in the precipitation operation is not particularly limited as long as it allows the compound (3) to precipitate as a solid and dissolves a small amount of remaining raw materials or by-products, and examples thereof include ether-based solvents, aliphatic hydrocarbon-based solvents, lower aliphatic acid ester-based solvents, alcohol-based solvents, and water. Lower aliphatic acid ester-based solvents are preferable, methyl acetate, ethyl acetate, propyl acetate, and isopropyl acetate are more preferable, and ethyl acetate is still more preferable.

Two or more of the solvents can be used in combination.

The temperature is not particularly limited as long as it is a temperature at which the compound (3) precipitates, and impurities such as remaining raw materials and by-products dissolve.

For example, when the compound (3) is a compound of Formula (3a), it can be obtained as a solid from its ethyl acetate solution by the precipitation operation and the solid-liquid separation.

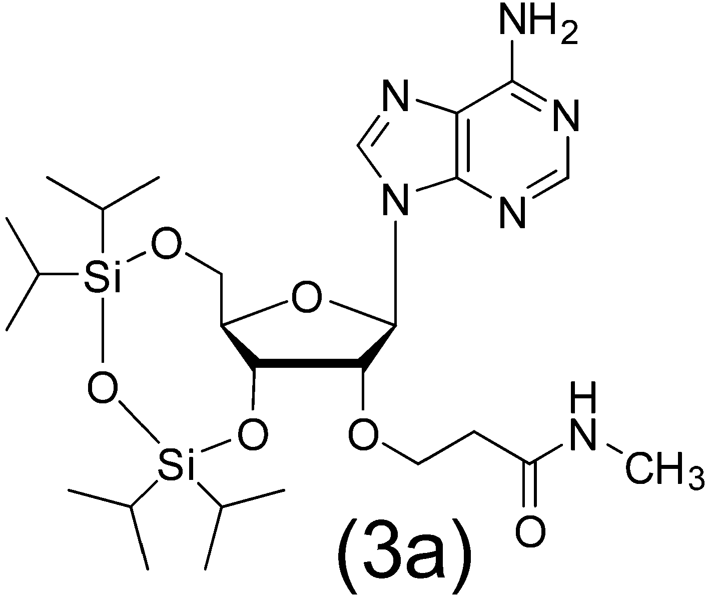

The stirring temperature is preferably -10 to 70°C, and more preferably 0 to 60°C.

Step (c): adenine protection step

The step (c) is a step of converting the compound (3) into the compound (4).

The compound (4) can be synthesized by protecting the 6-amino group of the compound (3).

As the protecting group for the 6-amino group of adenine, as described above, protecting groups commonly used in organic synthetic chemistry (for example, nucleic acid chemistry) can be used, and protection can be performed by, for example, the method described in Protective Groups in Organic Synthesis, Fourth edition, authored by T. W. Greene, John Wiley & Sons Inc. 2006. Acyl-based protecting groups such as an acetyl group, propionyl group, i-butyryl group, pivaloyl group, n-butyryl group, benzoyl group, and phenoxyacetyl group are preferable, an acetyl group, benzoyl group, i-butyryl group, and phenoxyacetyl group are more preferable, an acetyl group and benzoyl group are still more preferable, and an acetyl group is yet more preferable.

Protection of the 6-amino group of adenine with an acyl-based protecting group (for example, i-butyryl, acetyl, benzoyl, phenoxyacetyl) can be performed by reacting with an acyl halide (for example, isobutyryl chloride, acetyl chloride, benzoyl chloride, or phenoxyacetyl chloride) or an acid anhydride (for example, acetic anhydride) in a solvent in the presence of a base (for example, triethylamine, N,N-diisopropylethylamine, or pyridine). The solvent is not particularly limited as long as it effectively dissolves the compound (3) and allows the protection reaction to proceed smoothly, and ether-based solvents such as tetrahydrofuran, halogenated hydrocarbon-based solvents such as dichloromethane, lower aliphatic acid ester-based solvents such as ethyl acetate, and pyridine-based solvents such as pyridine are preferable, and pyridine is more preferable. The amount of the acyl halide or acid anhydride used with respect to 1 mol equivalent of the compound (3) is preferably 1 to 10 mol equivalents, more preferably 1 to 5 mol equivalents, and still more preferably 1 to 2 mol equivalents.

The temperature is preferably 0 to 100°C, more preferably 25 to 100°C, and still more preferably 80 to 100°C.

The reaction time is, for example, 0.5 to 48 hours, preferably 1 to 24 hours, and more preferably 1 to 8 hours.

The compound (4) obtained in the step (c) can be used in the next step (d) without purification.

### Step (d): deprotection step

The step (d) is a step of converting the compound (4) into the compound (5).

The compound (5) can be synthesized by deprotecting the 3'- and 5'-hydroxy groups of the compound (4).

The deprotection of the 3'- and 5'-hydroxy groups of the compound (4) can be performed, for example, under the conditions described in Protective Groups in Organic Synthesis, Fourth edition, authored by T. W. Greene, John Wiley & Sons Inc. 2006.

For example, the deprotection of the 3'- and 5'-hydroxy groups can be performed by reacting a reaction substrate with a fluorine compound (for example, ammonium fluoride, tetrabutylammonium fluoride, triethylamine trihydrofluoride, etc.) in a solvent. The solvent is preferably an alcohol-based solvent or an ether-based solvent, and more preferably tetrahydrofuran. The base itself can be used as the solvent. The amount of the fluorine compound used with respect to 1 mol equivalent of the compound (3) (with respect to the compound (2) when the compound (4) is not purified in the step (c)) is preferably 1 to 10 mol equivalents, more preferably 1 to 5 mol equivalents, and still more preferably 1 to 2 mol equivalents.

The reaction temperature is preferably -20°C to the solvent reflux temperature, more preferably 0 to 60°C, and still more preferably 20 to 40°C. The reaction time is, for example, 0.5 to 48 hours, preferably 1 to 36 hours, and more preferably 1 to 24 hours.

The compound (5) obtained in the step (d) can be used in the next step (e) without purification.

### Step (e): protection step of the 5'-hydroxy group

The step (e) is a step for converting the compound (5) into the compound (6).

The compound (6) can be synthesized by protecting the 5'-hydroxy group of the compound (5).

As the protection of the 5'-hydroxy group, as described above, protecting groups commonly used in organic synthetic chemistry (for example, nucleic acid chemistry) can be used, and protection can be performed by, for example, the method described in Protective Groups in Organic Synthesis, Fourth edition, authored by T. W. Greene, John Wiley & Sons Inc. 2006.

For example, a triarylmethyl-based protecting group (for example, trityl group, or 4,4'-dimethoxytrityl group) can be introduced into the 5'-hydroxy group by reacting a triarylmethylating agent (for example, trityl chloride, or 4,4'-dimethoxytrityl chloride) in a solvent in the presence of a base (for example, triethylamine, N,N-diisopropylethylamine, or pyridine). Any solvent that does not affect the reaction may be used, and halogenated hydrocarbon-based solvents such as dichloromethane and pyridine-based solvents such as pyridine are preferable, and pyridine is more preferable. Here, in this step, the pyridine-based solvent also functions as a base. The amount of the triarylmethylating agent used with respect to 1 mol equivalent of the compound (5) (with respect to the compound (2) when the compound (4) is not purified in the step (c) and the compound (5) is not purified in the step (d)) is preferably 1 to 10 mol equivalents, more preferably 1 to 5 mol equivalents, and still more preferably 1 to 2 mol equivalents.

The reaction temperature is preferably 0°C to the solvent reflux temperature, and more preferably 20 to 30°C.

The reaction time is, for example, 0.5 to 48 hours, preferably 0.5 to 24 hours, and more preferably 0.5 to 4 hours.

Step (f): Phosphoramidite formation step at the 3'-position of the hydroxy group.

The compound (7) can be synthesized by converting the 3'-hydroxy group of the compound (6) into a phosphoramidite.

Conversion of the 3'-hydroxy group into a phosphoramidite can be performed under acidic conditions (for example, 4,5-dicyanoimidazole, or 1H-tetrazole) using a phosphoramidite formation agent (for example, 2-cyanoethoxy N,N,N',N'-tetraisopropyl phosphorodiamidite, or 2-cyanoethoxy N,N-diisopropyl chlorophosphoramidite). Any solvent that does not affect the reaction may be used. Halogenated hydrocarbon-based solvents such as dichloromethane, lower aliphatic acid ester-based solvents such as ethyl acetate, nitrile-based solvents such as acetonitrile, and ether-based solvents such as 4-methyltetrahydropyran are preferable, and dichloromethane, ethyl acetate, 4-methyltetrahydropyran, and acetonitrile are more preferable. The amount of the phosphoramidite formation agent used with respect to 1 mol equivalent of the compound (6) is preferably 1 to 10 mol equivalents, more preferably 1 to 5 mol equivalents, and still more preferably 1 to 2 mol equivalents.

The reaction temperature is preferably 0 to 50°C, and more preferably 20 to 30°C.

The reaction time is, for example, 0.5 to 48 hours, preferably 1 to 24 hours, and more preferably 1 to 12 hours.

Use of diol silica gel instead of common silica gel in the column chromatography purification of compound (7) allowed reduction in the operation, materials, and time required for purification. Specifically, the developing solvent composition can be simplified, the total amount of the developing solvent can be reduced and/or the reproducibility of purification can be improved. The diol silica gel is a silica gel to which a diol group is bonded. Examples of diol groups include alkyl groups having two hydroxy groups such as a dihydroxypropyl group. The diol group is preferably a C₁₋₆ alkyl group having two hydroxy groups, more preferably a C₁₋₄ alkyl group having two hydroxy groups, and still more preferably a dihydroxypropyl group.

When a diol silica gel is used, purification can be performed without adding a tertiary amine (triethylamine, etc.) to a combination of a hydrocarbon-based solvent (hexane, heptane, etc.) and a lower aliphatic acid ester-based solvent (ethyl acetate, etc.) or a combination of a halogenated hydrocarbon-based solvent (chloroform, dichloromethane, etc.) and an alcohol-based solvent (methanol, etc.) as a developing solvent.

According to one embodiment of the present production method, the overall process from Step (a) to Step (f) allows a dramatic reduction in the labor required for purification by column chromatography, as compared to conventional methods. Typically, in a method for producing a 2'-O-2-(N-substituted carbamoyl)ethylated adenosine phosphoramidite using an adenosine compound in which the 3'- and 5'-hydroxy groups are silyl-protected as a starting material through a step (a) of performing a Michael addition reaction with an acrylic acid ester, a step (b) of amidating the ester moiety of a substituent introduced into the 2'-hydroxy group, a step (c) of protecting the 6-amino group of the adenine moiety, a step (d) of deprotecting the 3'- and 5'-hydroxy groups, a step (e) of trityl-protecting of the 5'-hydroxy group, and a step (f) of converting the 3'-hydroxy group into a phosphoramidite, when the intermediate obtained in the step (b) is purified by a precipitation operation and solid-liquid separation, the three steps (c) to (e) are performed consecutively, and purification by column chromatography after phosphoramidite formation in the step (f) is performed using a diol silica gel, which can reduce the complexity of the purification, it is possible to achieve the production method suitable for industrial production.

### [Examples]

The present invention will be described below in more detail with reference to examples, but the present invention is not limited to these examples.

In examples, "LC-MS" means liquid chromatography mass spectrometry, and "NMR" means nuclear magnetic resonance. "(v/v)" means (volume/volume).

In examples, purification by silica gel column chromatography was performed using Purif-Pack (registered trademark)-EX (SI-50 µm) (SHOKO SCIENCE) or CHROMATOREX DIOL MB100-40/75 (Fuji Silysia Chemical Ltd.).

¹H-NMR spectrum data were measured at 300 MHz (JNM-ECP300; JEOL Ltd., or JNM-ECX300; JEOL Ltd.), or 400 MHz (JNM-ECZ400S; JEOL Ltd.) using tetramethylsilane (0.0 ppm) as an internal standard. ³¹P-NMR spectrum data were measured at 162 MHz (JNM-ECZ400S; JEOL Ltd.) using trimethyl phosphate as an internal standard (3.5 ppm for DMSO-d6 and 3.0 ppm for CDCl₃) (see Spectrochimica Acta, Part A, vol. 55, pp. 1049-1057 (1999)). The J values were indicated in Hz, and the chemical shifts were indicated in ppm. "s" stands for singlet, "d" stands for doublet, "t" stands for triplet, "q" stands for quartet, "quint" stands for quintet, "dd" stands for doublet of doublets, "m" stands for multiplet, "brs" stands for broad singlet, "brm" stands for broad multiplet, "CDCl₃" stands for deuterated chloroform, and "DMSO-d₆" stands for deuterated dimethyl sulfoxide.

Unless otherwise specified, LC-MS data were measured using the ESI (electrospray ionization) method under the following conditions. "ESI⁺" means an ESI positive ion mode, and "ESI⁻" means an ESI negative ion mode.

In addition, unless otherwise specified, the LC purity was measured under the following conditions and calculated by the area percentage method.

### LC-MS analysis condition 1:

High performance liquid chromatography: HPLC commercially available from Waters Column: ACQUITY UPLC BEH C18 1.7 µM 2.1×50 mm; Waters
Column oven temperature: 40°C
Eluent: Solution A: 0.1% formic acid aqueous solution
Solution B: 0.1% formic acid acetonitrile solution

### Gradient condition:

Measurement was started at a flow rate of 0.6 mL/min and at a mixing ratio of the solution A and the solution B of 90/10, and the mixing ratio of the solution A and the solution B was then linearly changed to 10/90 over 3 minutes.

Subsequently, the mixing ratio of the solution A and the solution B was fixed at 10/90 for 0.7 minutes.

Subsequently, the mixing ratio of the solution A and the solution B was linearly changed to 90/10 and the flow rate was linearly changed to 0.8 mL/min for 0.1 minutes.

Subsequently, the mixing ratio of the solution A and the solution B was fixed at 90/10 for 1 minute.

### Detection wavelength: 254 nm

### LC-MS analysis condition 2:

High performance liquid chromatography: HPLC commercially available from Waters Column: ACQUITY UPLC BEH C18 1.7 µM 2.1×50 mm ; Waters
Column oven temperature: 40°C
Eluent: Solution A: 10 mM ammonium bicarbonate aqueous solution
Solution B: 10 mM ammonium bicarbonate aqueous solution/acetonitrile=1:9

### Gradient condition:

Measurement was started at a flow rate of 0.6 mL/min and at a mixing ratio of the solution A and the solution B of 90/10, and the mixing ratio of the solution A and the solution B was then linearly changed to 10/90 over 3 minutes.

Subsequently, the mixing ratio of the solution A and the solution B was fixed at 10/90 for 0.7 minutes.

Subsequently, the mixing ratio of the solution A and the solution B was linearly changed to 90/10 and the flow rate was linearly changed to 0.8 mL/min for 0.1 minutes.

Subsequently, the mixing ratio of the solution A and the solution B was fixed at 90/10 for 1 minute.
Detection wavelength: 254 nm

### [Example 1]

### Production of 2'-O-[2-(N-methylcarbamoyl)ethyl]-3',5'-O-(1,1,3,3-tetraisopropyldisiloxane-1,3-diyl)ad enosine

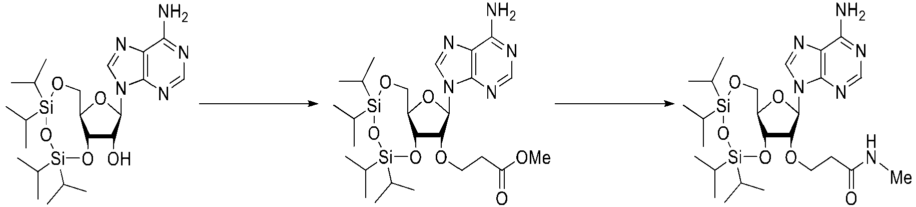

3',5'-O-(1,1,3,3-tetraisopropyldisiloxane-1,3-diyl)adenosine (19.0 g, 37.3 mmol) known in the document was added to tert-butyl alcohol (38.0 g) and methyl acrylate (48.2 g, 559 mmol). After the reaction mixture was cooled to 0°C, a 1 M potassium tert-butoxide tetrahydrofuran solution (18.6 mL, 18.6 mmol) was added dropwise over 18 minutes. After the mixture was stirred at 0°C for 30 minutes, acetic acid (1.59 g, 26.1 mmol) was added, and the mixture was stirred at room temperature for 15 minutes. tert-Butyl methyl ether (190 g) and 5 wt% aqueous sodium chloride solution (95.1 g) were added to the reaction mixture for extraction. The obtained organic layer was dried over magnesium sulfate, the solids were removed by filtration, and the filtrate was then washed with tert-butyl methyl ether (38.0 g). The filtrates were combined and concentrated under reduced pressure to about 76.0 g. Tetrahydrofuran (190 g) was added to the concentrated solution, which was then concentrated under reduced pressure to 45.8 g. Tetrahydrofuran (95.2 g) was added to the concentrated solution, which was then concentrated under reduced pressure to 40.1 g. Tetrahydrofuran (4.5 g) was added to the concentrated solution to obtain a tetrahydrofuran solution (44.6 g) of 2'-O-[2-(methoxycarbonyl)ethyl]-3',5'-O-(1,1,3,3-tetraisopropyldisiloxane-1,3-diyl)aden osine.
LC/MS: analysis condition 1, retention time = 2.95 minutes
LC/MS (ESI+) m/z; 596.7 [M+H]⁺
LC purity 77%

Then, to 35.2 g of the solution was added 40 wt% methylamine-methanol solution (30 mL, 294 mmol) at room temperature. After the mixture was stirred at room temperature for 15 hours 20 minutes, ethyl acetate (175 g) and water (52.5 g) were added to the reaction solution for extraction. The obtained organic layer was dried over magnesium sulfate, the solid components were removed by filtration, and the filtrate was then washed with ethyl acetate (35.3 g). The filtrates were combined and concentrated under reduced pressure to 54.3 g. Ethyl acetate (87.7 g) was added to the concentrated solution, which was then concentrated under reduced pressure to 43.9 g. Ethyl acetate (87.6 g) was added to the concentrated solution, which was then concentrated under reduced pressure to 59.2 g. Ethyl acetate (2.4 g) was added to the concentrated solution, the mixture was then stirred at 60°C for 1 hour, heating was stopped, and the mixture was stirred for 16 hours. The precipitated solid was filtered, and the filtrate was washed twice with ethyl acetate (15.8 g). The obtained solid was dried under reduced pressure at 50°C, and a title compound was obtained as a white solid (5.61 g, yield 32%).
LC/MS: analysis condition 1, retention time = 2.86 minutes
LC/MS (ESI+) m/z; 595.3 [M+H]⁺
LC purity 93%
¹H-NMR (CDCl₃, 300 MHz) δ: 0.99-1.11 (28H, m), 2.42-2.50 (1H, m), 2.64-2.74 (1H, m), 2.83 (3H, d, J = 4.8 Hz), 4.00-4.30 (6H, m), 4.61-4.66 (1H, m), 5.67 (2H, brs), 6.02 (1H, s), 8.16 (1H, s), 8.32 (1H, s)

### [Example 2]

### Production of 6-N-acetyl-5'-O-(4,4'-dimethoxytrityl)-2'-O-[2-(N-methylcarbamoyl)ethyl]adenosine

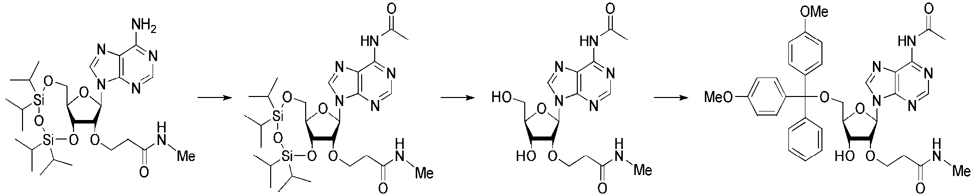

Acetic anhydride (12.9 g, 126 mmol) was added at 95°C to a pyridine (100 g) solution of 2'-O-[2-(N-methylcarbamoyl)ethyl]-3',5'-O-(1,1,3,3-tetraisopropyldisiloxane-1,3-diyl)ad enosine (50.0 g, 84.1 mmol) obtained in Example 1. The reaction mixture was stirred at 95°C for 4 hours, water (0.910 g, 50.6 mmol) was then added, and the mixture was stirred at 95°C for 2 hours and 10 minutes. After cooling to room temperature, ethyl acetate (450 g) was added, and the mixture was washed twice with water (250 g). The organic layer was concentrated under reduced pressure to 96.0 g to obtain an ethyl acetate solution of 6-N-acetyl-2'-O-[2-(N-methylcarbamoyl)ethyl]-3',5'-O-(1,1,3,3-tetraisopropyldisiloxane -1,3-diyl)adenosine.
LC/MS: analysis condition 2, retention time = 3.26 minutes
LC/MS (ESI+) m/z; 637.7 [M+H]⁺
LC purity 92%

Then, pyridine (100 g) and triethylamine trihydrofluoride (13.6 g, 84.1 mmol) were added to this solution at room temperature, and the reaction mixture was stirred at room temperature for 5 hours. Methoxytrimethylsilane (17.5 g, 168 mmol) was added at room temperature, and the mixture was stirred at room temperature for 1 hour to obtain a pyridine solution (224 g) of 6-N-acetyl-2'-O-[2-(N-methylcarbamoyl)ethyl]adenosine.
LC/MS: analysis condition 2, retention time = 0.24 minutes
LC/MS (ESI+) m/z; 395.5[M+H]⁺
LC purity 97%

In addition, a pyridine solution (220 g) of 6-N-acetyl-2'-O-[2-(N-methylcarbamoyl)ethyl]adenosine was concentrated under reduced pressure to 98.3 g, 323 g of pyridine was added, the mixture was concentrated again under reduced pressure to 72.0 g, and pyridine (98.4 g) was added. A solution of 4,4'-dimethoxytrityl chloride (42.0 g, 124 mmol) in dichloromethane (130 g) was added dropwise to this solution at room temperature for 27 minutes. The reaction mixture was stirred at room temperature for 1 hour and 30 minutes, water (0.756 g, 41.2 mmol) was then added, and the mixture was stirred for 30 minutes. The reaction solution was concentrated under reduced pressure to 244 g, ethyl acetate (323 g) was then added, and the mixture was washed with water (323 g) and 9 wt% aqueous sodium chloride solution (355 g). The organic layer was concentrated under reduced pressure, and the residue was then purified by silica gel column chromatography (chloroform/methanol: trimethylamine (2:1) = 95/5(v/v)). Eluted fractions of 6-N-acetyl-5'-O-(4,4'-dimethoxytrityl)-2'-O-[2-(N-methylcarbamoyl)ethyl]adenosine were collected and concentrated under reduced pressure to 96.0 g. Ethyl acetate (981 g) was added to the concentrated solution, which was then concentrated under reduced pressure to 140 g, ethyl acetate (981 g) was added again, and the mixture was concentrated under reduced pressure to 106 g. Ethyl acetate (133 g) was added to the concentrated solution to obtain an ethyl acetate solution (43.1 g, quantitative yield 75%, 20 wt% solution) of 6-N-acetyl-5'-O-(4,4'-dimethoxytrityl)-2'-O-[2-(N-methylcarbamoyl)ethyl]adenosine.
LC/MS: analysis condition 2, retention time = 2.48 minutes
LC/MS (ESI+) m/z; 697.3 [M+H]⁺
LC purity 99%

### [Example 3]

### Production of 6-N-acetyl-5'-O-(4,4'-dimethoxytrityl)-2'-O-[2-(N-methylcarbamoyl)ethyl]adenosine-3'-(2-cyanoethyl-N,N-diisopropylphosphoramidite)

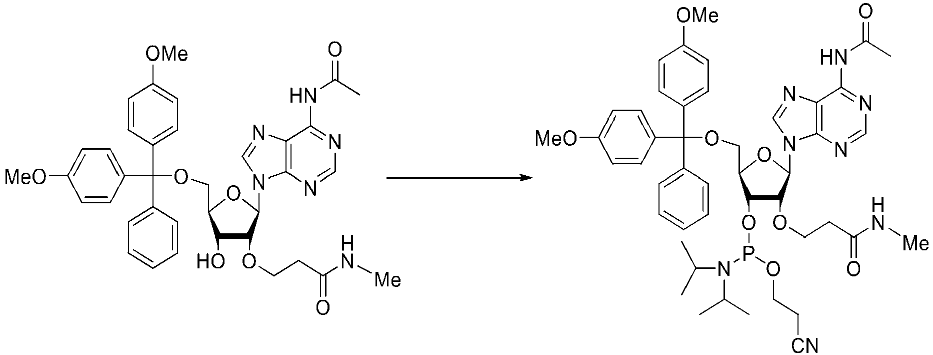

2-Cyanoethoxy N,N,N',N'-tetraisopropyl phosphorodiamidite (15.1 g, 50.2 mmol) was added to an ethyl acetate solution (solution weight 125 g) of 6-N-acetyl-5'-O-(4,4'-dimethoxytrityl)-2'-O-[2-(N-methylcarbamoyl)ethyl]adenosine obtained in Example 2. A solution of 4,5-dicyanoimidazole (2.97 g, 25.1 mmol) in 4-methyltetrahydropyran (42.7 g) was added dropwise to this reaction mixture at room temperature over 11 minutes. The reaction mixture was stirred at room temperature for 1 hour 20 minutes, ethyl acetate (101 g) was then added, and the mixture was washed three times with a 1.4 wt% potassium bicarbonate aqueous solution (507 g). The obtained organic layer was dried over magnesium sulfate, the solid components were then removed by filtration, and the filtrate was then washed with ethyl acetate (50.3 g). The filtrates were combined and concentrated under reduced pressure. The residue was purified by column chromatography (heptane/ethyl acetate = 1/1-0/1 (v/v)) using diol silica gel, and a title compound was obtained as a white amorphous solid (21.1 g, yield 65%).
¹H-NMR (DMSO-d₆, 400 MHz) δ: 1.02 (3.0H, d, J = 6.6 Hz), 1.12-1.16 (8.2H, m), 2.23-2.32 (5.0H, m), 2.45 (1.5H, d, J = 4.8 Hz), 2.46 (1.5H, d, J = 4.8 Hz), 2.61 (0.8H, t, J = 5.9 Hz), 2.73-2.86 (1.0H, m), 3.20-3.36 (2.0H, m), 3.49-3.88 (12.0H, m), 4.19 (0.5H, ddd, J = 5.0, 4.3, 4.3 Hz), 4.24 (0.4H, ddd, J = 5.0, 4.3, 4.3 Hz), 4.70( 0.5H, ddd, J = 5.0, 5.0, 10.0 Hz), 4.73 (0.5H, ddd, J = 5.0, 5.0, 10.0 Hz), 4.88 (0.5H, dd, J = 5.1, 5.0 Hz), 4.89 (0.5H, dd, J = 5.1, 5.0 Hz), 6.11 (0.4H, d, J = 5.1 Hz), 6.14 (0.5H, d, J = 5.1 Hz), 6.76-6.85 (4.0H, m), 7.14-7.26 (7.0H, m), 7.30-7.36 (1.9H, m), 7.67 (0.9H, q, J = 4.6 Hz), 8.54 (0.5H, s), 8.57 (0.4H, s), 8.60 (0.4H, s), 8.61 (0.5H, s), 10.74 (1.9H, s).
³¹P-NMR(DMSO-d₆, 162 MHz) δ 15.018, 150.33
LC/MS: analysis condition 2, retention time = 3.18, 3.32 minutes
LC/MS (ESI+) m/z; 897.3 [M+H]⁺
LC purity 98%

### INDUSTRIAL APPLICABILITY

Accodring to the present invention, it is possible to provide novel methods for producing a 2'-O-modified adenosine phosphoramidite and an intermediate thereof.

## Claims

1. A method for producing a compound of Formula (2), **characterized by** comprising adding a catalytic amount of an alkali metal tertiary alkoxide to a solution or suspension of a mixture of a compound of the following Formula (1) and an acrylic acid ester of Formula (8) in a tertiary alcohol-based solvent: 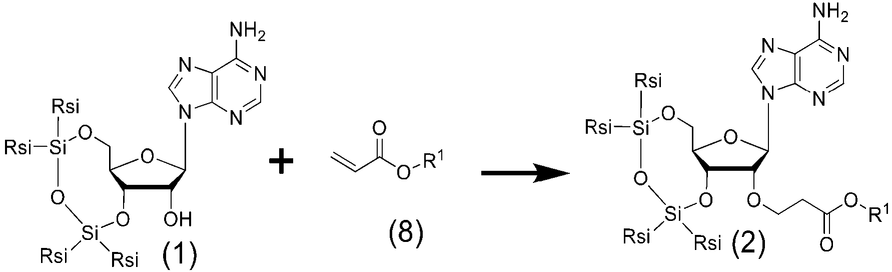 [wherein R¹ is a C₁₋₄ alkyl group which arbitrarily has a substituent or a C₇₋₁₆ aralkyl group which arbitrarily has a substituent, and Rsi's are each independently a C₁₋₆ alkyl group or a phenyl group].

2. The method according to claim 1, wherein R¹ is a methyl group, an ethyl group, a trifluoroethyl group, or a benzyl group.

3. The method according to claim 1, wherein R¹ is a methyl group.

4. The method according to claim 1, wherein the tertiary alcohol-based solvent is tert-butanol.

5. The method according to claim 1, wherein the number of equivalents of the alkali metal tertiary alkoxide per 1 equivalent of the compound of Formula (1) is 0.1 equivalents to 0.8 equivalents.

6. The method according to claim 1, wherein the alkali metal tertiary alkoxide is

7. A method for producing a compound of the following Formula (3), comprising: a step (b1) of reacting a compound of the following Formula (2) with a primary amine of the following Formula (9) to produce a compound of the following Formula (3); and a step (b2) of purifying the compound of Formula (3) by a precipitation operation and solid-liquid separation: 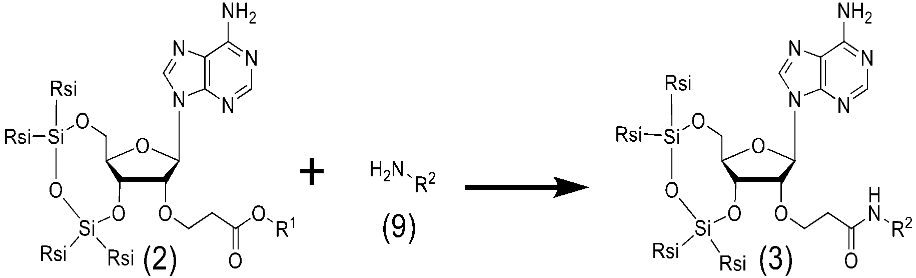 [in Formula (2), R¹ is a C₁₋₄ alkyl group which arbitrarily has a substituent or a C₇₋₁₆ aralkyl group which arbitrarily has a substituent,
in Formulae (2) and (3), Rsi's are each independently a C₁₋₆ alkyl group or a phenyl group, and
in Formulae (3) and (9), R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent].

8. The method according to claim 7, wherein R¹ is a methyl group, an ethyl group, a trifluoroethyl group, or a benzyl group.

9. The method according to claim 7, wherein R¹ is a methyl group.

10. The method according to claim 7, wherein R² is a methyl group.

11. A method for producing a compound of the following Formula (6), comprising:
a step (c) of converting a compound of the following Formula (3) into a compound of the following Formula (4);
a step (d) of converting the compound of Formula (4) into a compound of the following Formula (5); and
a step (e) of converting the compound of Formula (5) into a compound of the following Formula (6): 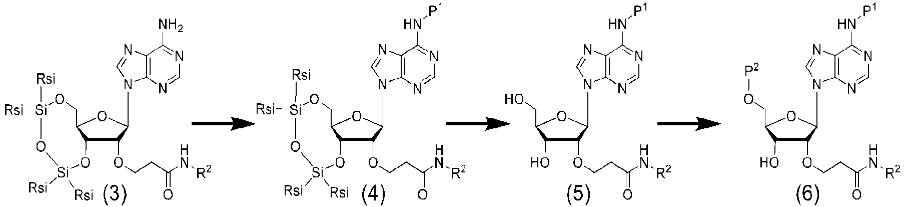
[in Formulae (3), (4), (5), and (6), R² is a C₁₋₆ alkyl group which arbitrarily has a substituent, a C₇₋₁₆ aralkyl group which arbitrarily has a substituent, a C₂₋₆ alkenyl group which arbitrarily has a substituent, a C₂₋₆ alkynyl group which arbitrarily has a substituent, or a C₆₋₁₄ aryl group which arbitrarily has a substituent,
in Formulae (3) and (4), Rsi's are each independently a C₁₋₆ alkyl group or a phenyl group,
in Formulae (4), (5), and (6), P¹ is a protecting group for an amino group, and
in Formula (6), P² is a protecting group for a hydroxy group].

12. The method according to claim 11, wherein R² is a methyl group.

13. The method according to claim 11, wherein P¹ is an acetyl group, a propionyl group, an i-butyryl group, a pivaloyl group, an n-butyryl group, a benzoyl group, or a phenoxyacetyl group.

14. The method according to claim 11, wherein P¹ is an acetyl group.

15. The method according to claim 11, wherein P² is a 4,4'-dimethoxytrityl group.

16. The method according to claim 11, wherein the process proceeds to a next step without purification by column chromatography in the step (c) and the step (d), and purification by column chromatography is performed in the step (e).

17. A method for purifying a compound of the following Formula (7) by column chromatography using a diol silica gel: 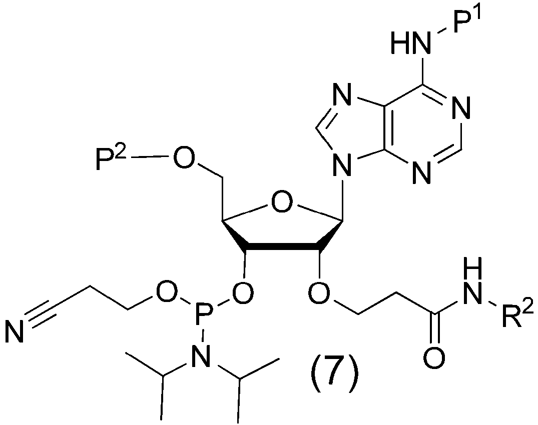 [wherein R² is a C₁₋₆ alkyl group which arbitrarily has a substituent, a C₇₋₁₆ aralkyl group which arbitrarily has a substituent, a C₂₋₆ alkenyl group which arbitrarily has a substituent,
a C₂₋₆ alkynyl group which arbitrarily has a substituent, or a C₆₋₁₄ aryl group which arbitrarily has a substituent, P¹ is a protecting group for an amino group, and P² is a protecting group for a hydroxy group].

18. The method according to claim 17, wherein R² is a methyl group.

19. The method according to claim 17, wherein P¹ is an acetyl group, a propionyl group, an i-butyryl group, a pivaloyl group, an n-butyryl group, a benzoyl group, or a phenoxyacetyl group.

20. The method according to claim 17, wherein P¹ is an acetyl group.

21. The method according to claim 17, wherein P² is a 4,4'-dimethoxytrityl group.

22. A method for producing a compound of Formula (7), comprising:
producing a compound of Formula (6) by the method according to claim 11;
producing the compound of Formula (7) by converting the compound of Formula (6) into a phosphoramidite; and
purifying the compound of Formula (7) by the method according to claim 17.

23. The method according to claim 22, wherein the compound of Formula (3) is produced by the method according to claim 7.

24. The method according to claim 23, wherein the compound of Formula (2) is produced by the method according to claim 1.
